(19) Europäisches Patentamt
European Patent Office
Office européen des brevets

(11) Publication number: **0 439 450 B1**

(12) # EUROPEAN PATENT SPECIFICATION

(45) Date of publication of patent specification: **23.12.92**

(51) Int. Cl.⁵: **A61K 31/155**, //(A61K31/155, 31:055)

(21) Application number: **88906943.1**

(22) Date of filing: **28.07.88**

(86) International application number: **PCT/GB88/00630**

(87) International publication number: **WO 89/00850 (09.02.89 89/04)**

(54) **PHARMACEUTICAL COMPOSITION.**

(30) Priority: **29.07.87 GB 8718026**

(43) Date of publication of application: **07.08.91 Bulletin 91/32**

(45) Publication of the grant of the patent: **23.12.92 Bulletin 92/52**

(84) Designated Contracting States: **AT BE CH DE FR GB IT LI NL SE**

(56) References cited:
**DE-A- 2 351 386**

**UNLISTED DRUGS, vol. 24, no. 6, June 1972, Chatham, NJ (US); p. 81k "P-300"&NUM;**

**UNLISTED DRUGS, vol. 32, no. 11, November 1980, Chatham, NJ (US); p. 1741 "Rhulicaine"&NUM;**

**UNLISTED DRUGS, vol. 33, no. 5, May 1981, Chatham, NJ (US); p. 72h "Eubos"&NUM;**

(73) Proprietor: **SMITH & NEPHEW plc
2 Temple Place Victoria Embankment
London WC2R 3BP(GB)**

(72) Inventor: **RICHARDSON, Mark, Christopher 4
St. John's Close
Great Chesterford
Saffron Walden Essex(GB)**

(74) Representative: **Cole, William Gwyn, Dr.
Smith and Nephew Research Limited Gilston Park
Harlow Essex CM20 2RO(GB)**

EP 0 439 450 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to pharmaceutical compositions which are suitable for topical application to the skin for the prevention of or in the treatment of bacterial infections and which contain a hydroxy halogenated diphenyl derivative particularly triclosan and a diamidine derivative particularly dibromopropamidine.

Compounds of formula (I).

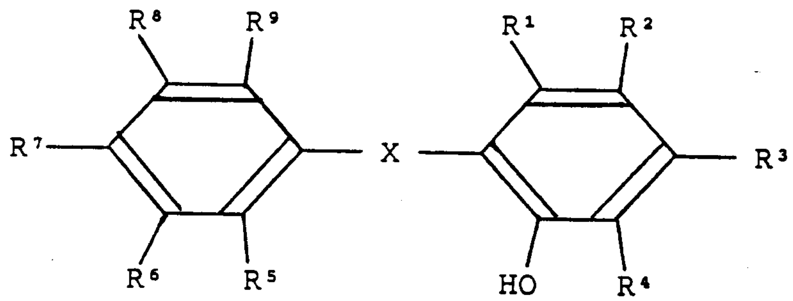

(I)

wherein X is 0 or $CH_2$ and each of $R^1$ to $R^9$ may be hydrogen, hydroxyl or halogen are known to have excellent bactericidal activity against many gram positive and gram negative bacteria and are fungicidal and algicidal and also have good skin substantivity. However they show low activity against strains of Pseudomonas, Proteins and Serratia.

Compounds of formula (II)

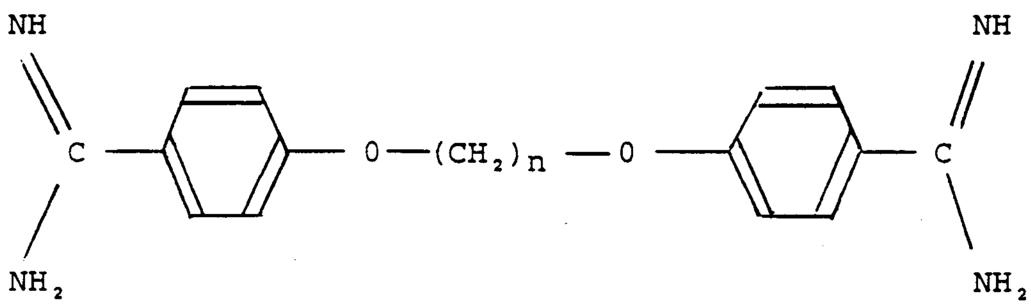

(II)

wherein n is an integer from 1 to 6 and each phenyl group may be further substituted by halogen are also known to have bactericidal and fungicidal activity but again a low activity is observed against certain organisms including strains of Pseudomonas, Proteins and Serratia.

It has been found that by admixing compounds of formula (I) and (II) in a pharmaceutical composition very desirable enhanced antibacterial activity is shown against strains of Pseudomonas, Proteins and Serratia which has hitherto not been shown. The discovery of this unexpected synergism in such a combination is useful, it enables lower concentrations of the ingredients to be used to give a desired antimicrobial effect and it allows a new combination of antibacterial agents to be used when otherwise the growth of Pseudomonas might be encouraged by the use of either antibacterial agent on its own.

Accordingly in one aspect the present invention provides a pharmaceutical composition suitable for topical application which comprises hydroxy halogenated diphenyl derivative of Formula (I)

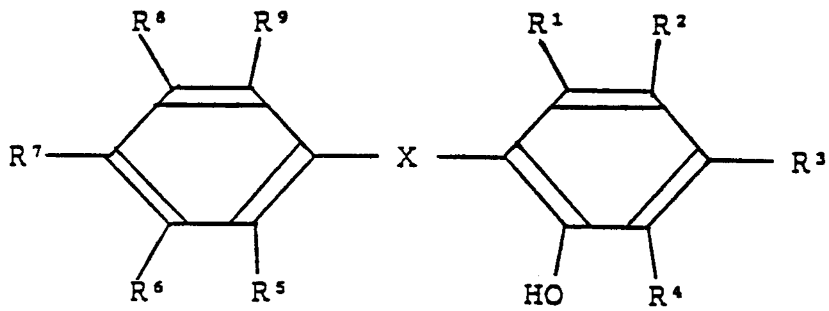

(I)

wherein X is O or $CH_2$ and each of $R^1$ to $R^9$ may be hydrogen, hydroxyl or halogen provided at least one of $R^1$ to $R^9$ is halogen and a diamidine of Formula (II)

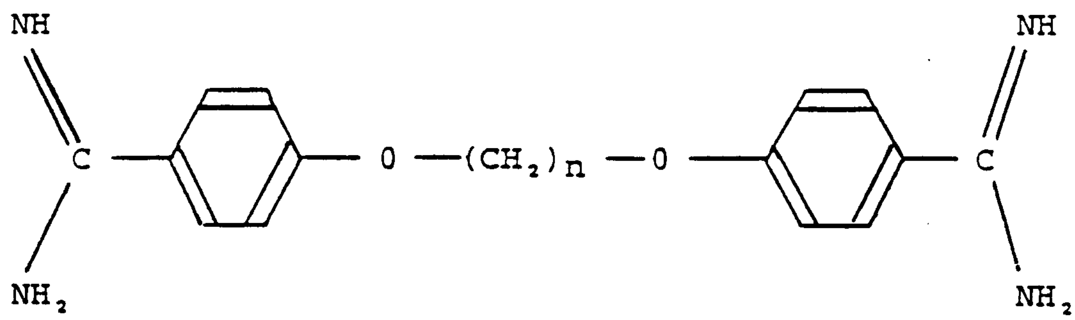

(II)

or a pharmaceutically-acceptable salt thereof, wherein n is an integer from 1 to 6 and each phenyl group may be further substituted by halogen; and a pharmaceutically acceptable carrier therefor.

In the definitions given above the term halogen is generic to fluoro, chloro, bromo and iodo.

Aptly when X is 0 in formula (I), $R^1$ to $R^9$ may be hydrogen or halogen, especially chloro and more aptly at least two of $R^1$ to $R^9$ are chloro. In a preferred compound of formula (I) $R^3$, $R^5$ and $R^7$ are each chloro and $R^1$, $R^2$, $R^4$, $R^6$, $R^8$, $R^9$ are each hydrogen. Thus a preferred compound of formula (I) is 2, 4, $4^1$-trichloro $2^1$-hydroxydiphenyl ether (triclosan).

Aptly when X is $CH_2$ in formula (I) and $R^5$ is hydroxy, the remaining substituents may be hydrogen or halogen especially chloro or bromo, and preferably there are at least two halogen substituents to each phenyl ring. In a preferred compound of formula (I) $R^1$, $R^2$, $R^4$, $R^6$, $R^8$ and $R^9$ are each chloro and $R^5$ is hydroxy. Thus a second preferred compound of formula (I) is 2, $2^1$ dihydroxy-3, $3^1$, 5, $5^1$, 6, $6^1$-hexachlorodiphenylmethane (hexachlorophane).

An apt diamidine of formula (II) may have unsubstituted phenyl groups and n is an integer equal to 3. Thus one preferred diamidine of formula (II) is 4, $4^1$ -(trimethylenedioxy) dibenzamidine hereafter known as propamidine. A second apt diamidine of formula (II) may have unsubstituted phenyl groups and n is an integer equal to 6. Thus a second preferred diamidine of formula (II) is hexamidine. However in a particularly preferred compound each phenyl group (in formula II) is substituted by one halogen atom especially a bromine atom. Therefore a particularly preferred diamidine of formula (II) is dibromopropamidine. Diamidines are capable of forming salts with organic and inorganic acids. Thus diamidines of formula (II) may be present as a pharmaceutically acceptable salt. A particularly preferred salt is the isoethionate that is the di(2-hydroxy ethane sulphate).

Thus in a preferred aspect the present invention provides a pharmaceutical composition suitable for topical application which comprises 2, 4, $4^1$ trichloro-$2^1$-hydroxydiphenyl ether and dibromopropamidine isoethionate together with a pharmaceutically acceptable carrier therefor.

In a second preferred aspect the present invention provides a pharmaceutical composition suitable for topical application which comprises 2, $2^1$-dihydroxy - 3, $3^1$, 5, $5^1$, 6, $6^1$-hexachlorodiphenylmethane and dibromopropamidine isoethionate together with a pharmaceutically acceptable carrier therefor.

By topical administration it is meant to include as well as application to lesions of the skin such as wounds, burns, surgical trauma and general skin infections, but also administration to the vagina, rectum and other mucous membranes and also employment in skin preparations such as antiseptic solutions, peritoneal dialysis washout solutions, preoperative skin preparations, wound cleansing solutions and film-forming compositions for administration to broken or intact skin such as that beneath a so called IV dressing or in the treatment of acne.

Suitably, the amount of hydroxy halogenated diphenyl derivative of formula (I) which may be present in the compositions of the present invention may be from 0.1 to 10% by weight of the composition, more suitably will be from 0.2 to 5% by weight and preferably will be from 0.5 to 3.0% by weight for example 0.5%, 1%, 1.5%, 2.0% and 2.5%.

In compositions which are applied to intact skin the concentration of the hydroxy halogenated derivative of diphenylmethane or diphenyl ether is generally in the range 0.05 to 2% by weight of the composition and in the range 0.05 to 0.2% by weight if it is desired to prevent a high but not a normal growth of bacteria. In the treatment of infections the higher concentrations up to about 2% of the diphenyl derivative may be used.

Suitably the amount of diamidine compound which may be present in the compositions of the present invention may be from 0.01 to 1% by weight of the composition of the diamidine compound, more suitably will be from 0.05 to 0.5% by weight and preferably will be from 0.1 to 0.3% by weight, for example 0.10%, 0.15%, 0.2%, 0.25% and 0.3%.

Suitably the ratio by weight a hydroxy halogenated diphenyl derivative of formula (I) to diamidine of formula (II) in the compositions of the present invention will be in the range from 25:1 to 1:25, more suitably 20:1 to 1:20 and preferably from 15:1 to 1:15 for example 10:1, 2:1, 1:2 and 1:10.

The pharmaceutical compositions of the present invention are suitable for topical treatment of burns, ulcers and other skin lesions exposed to the risk of infection.

Aptly the compositions of the present invention may also include chelating agents such as ethylene diaminetetraacetic acid (EDTA) or a salt thereof. Suitably the salt is the salt of an alkali metal and is preferably a sodium salt. EDTA may be included as its mono-,di-,tri- or tetra-basic salt but in general the di- or tri-basic salts are preferred.

Suitably the compositions may contain from 0.01 to 5% of EDTA or a salt thereof and more suitably 0.05 to 2.5%, most suitably 0.1 to 2.0% and preferably 0.5 to 1.5%.

Suitable forms of the topical composition of this invention include aqueous solutions, aqueous-alcohol solutions, ointments, gels, oily suspensions, solid forms, for example tablets, powder, granules, pessaries and suppositories emulsions, lotions and films. The antibacterial agents may also be incorporated into dressings such as those described in European Patents Nos. 59049, 59048, 107915, 123485 and 122085, 256893 and into adhesives used on polymeric film wound dressings and drapes.

A topically administrable composition of the invention may be in the form of an ointment. This may conveniently have a hydrophilic ointment base such as an oil-in-water emulsion. Suitable ointment bases are described in Chapter 87 Ointments:

Emulsion Bases in Remingtons Pharmaceutical Sciences, 15th Ed. 1975, pages 1532-34. Also suitable ointment bases include those described in British Patent No. 1240545 as suitable for use with silver sulphadiazine and which is incorporated herein by cross reference.

A particularly suitable ointment base is therefore an oil-in-water emulsion containing from 0 to 25% of petrolatum of liquid paraffin, 2 to 20% of a fatty alcohol, 0 to 12% of an emulsifying agent, up to 10% of non-ionic surfactant and 5 to 25% of a polyhydric alcohol and the balance to 100% being water such as deionised or distilled water. Aptly the fatty alcohols are those conventionally used in ointments and are water insoluble. Suitable alcohols include stearyl alcohol, cetyl alcohol, lauryl alcohol and myristyl alcohol. Suitably the emulsifying agent is a glyceryl fatty acid ester and is preferably glyceryl monostearate. Suitable non-ionic surfactants include the polyoxyethylated sorbitan fatty acid esters and sorbitan fatty acid esters. An emulsifying wax may be used in place of both or part of both of the fatty alcohol and non-ionic surfactant. The polyhydric alcohol acts as a humectant and suitable alcohols include propylene glycol, sorbitol or glycerin or mixture thereof.

In a second aspect the compositions of the present invention will be in the form of an aqueous gel. Suitable gelling agents include polyoxyethylene-polyoxypropylene diol block copolymers, polyacrylic acid lightly cross-linked with triallyl sucrose which has been neutralised using an alkali metal hydroxide, cellulosic derivatives such as carboxymethyl cellulose, hydroxymethyl cellulose and natural gums. A preferred group of gelling agents are the polyoxyethylene-polyoxypropylene diol block copolymers which are commercially availabe as the Pluronics from BASF-Wyandotte. (Pluronic is a registered trade mark of BASF-Wyandotte).

Suitable gel forming block copolymers of polyoxyethylene-polyoxypropylene will have a molecular weight from 4,600 to 13,500 (approximately) and will be present in the gel in an amount from 50% for the lower molecular weight copolymers to 20% for the higher molecular weight copolymers, so that the gel when applied topically is neither too stiff nor too fluid. Typically the gels are formed by mixing together the copolymer and water to form an aqueous solution at a temperature of 2°C and adding a compound of formula (I) and (II) and then allowing the solution to gel as it warms to ambient temperature. Suitable Pluronics are those designated as F108, F127 and P105.

In a further aspect the composition of the present invention will be in the form of a hydrophobic ointment. Suitably hydrophobic ointments are those which are formed from white or yellow soft paraffin or a mixture of such with liquid paraffin. A preferred ointment base comprises a mixture of white soft paraffin and liquid paraffin in a ratio of 5:1 to 1:1. However, in general terms aqueous based systems will be preferred.

The hydrophobic ointment base may also contain non-ionic surfactants such as polyoxyethylated sorbitan fatty acid esters and sorbitan fatty acid esters. The presence of non-ionic surfactants increases the miscibility of the ointment with wound fluid and aids release of the medicament. Suitably the non-ionic surfactant will be present in an amount from 0.1 to 0.5%. Preferably the non-ionic surfactant is 0.1% or polyoxyethylene sorbitan trioleate and 0.1% sorbitan monopalmitate.

A more hydrophilic ointment may contain from 1 to 5% of a non-ionic surfactant. Suitable ointments are described in for example British Patent No. 1599159.

The compositions of this invention may comprise detergent compositions such as pre-surgical scrubbing compositions which are widely employed in the medical field. These compositions are usually in liquid form and contain a detergent such as a potassium soap, sorbitan monooleates, polyoxyethylated sorbitan monooleate and the corresponding lauryl derivatives. A triethanolamine lauryl sulphate or a sodium lauryl ethoxysulphate may also be included.

Other ingredients may be included in detergent compositions of the present invention including ethylenediamine tetra acetic acid (EDTA) and salts thereof and in particular the disodium salt of EDTA. Suitably a composition may contain from 0.05 to 2.5% of the disodium salt of EDTA more suitably 0.1 to 2.0% and preferably 0.5 to 1.5%

Detergent compositions may also contain alkanols such as ethanol, propanol or isopropanol. These compounds are commonly used in preoperative scrubs and skin prepping solutions. The alkanol may also facilitate the solubilisation of the diphenylether in the composition.

The combination may also be present in a pessary. Thus in a further aspect of the invention the composition is in the form of a pessary which contains hydroxy halogenated diphenyl derivative of formula (I) and diamidine compound of formula (II) or a pharmaceutically acceptable acid addition salt thereof. Pessaries are formed in a conventional manner including mixing a molten wax which melts at body temperature with the active ingredients and forming in a mould.

The pharmaceutical composition of the present invention may be used in a method of treatment which comprises applying topically a pharmaceutical composition which comprises hydroxy halogenated diphenyl derivative of formula (I) and a diamidine compound of formula (II) or a pharmaceutically acceptable acid addition salt thereof together with a pharmaceutically acceptable carrier therefor.

The method is applicable to treatment of lesions of the skin as hereinbefore defined, general skin infections and infections associated with the mucous membranes such as the vagina or rectum. Also the method is applicable on intact skin when the compositions are used for example as a surgical scrub, a preoperative scrub or a peritoneal dialysis washout solution.

The active ingredients may be incorporated into the composition by such conventional methods as mixing with the carrier, for example hydroxy halogenated diphenyl derivative of formula (I) and the diamidine compound may be added to an ointment base and stirred until a homogenous mixture is formed.

The percentage terms herein are expressed as a weight/weight basis.

From the foregoing it is clear that in a favoured aspect the present invention comprises a pharmaceutical composition adapted for topical administration for the treatment of burns, ulcers and other skin lesions exposed to the risk of infection which comprises from 0.5 to 3% of hydroxy halogenated diphenyl derivative of formula (I) and 0.1 to 0.3% of a diamidine of formula (II) or pharmaceutically acceptable salt thereof, 15 to 25% of liquid paraffin, 7 to 15% polyhydric alcohol, 4 to 8% of stearyl alcohol, 4 to 8% of glyceryl monostearate, 2 to 6% of a non-ionic surfactant and water to adjust the weight to 100%.

Pharmaceutical compositions of the present invention can therefore be used in a method of treating burns, ulcers, or other skin lesions such as acne which comprises applying topically thereto a pharmaceutical composition comprising an antimicrobially effective amount of hydroxy halogenated diphenyl derivative of formula (I) and diamidine of formula (II) as hereinbefore described together with a topically acceptable carrier therefor.

In another aspect the composition of the present invention may additionally contain other medicaments which are active when applied topically, for example antimicrobial agents and anti-inflammatories.

The compositions of the present invention may be sterilised prior to use. This may be achieved by means including (a) preparing the composition from presterilised components which are mixed under aseptic conditions and filled into presterilised packaging (b) sterilising the composition during preparation that is usually by heating then cooling and filling aseptically into presterilised packaging. This is the preferred means particularly for ointments. (c) sterilising the filled package containing the composition by conventional means and (d) aqueous solutions may be sterilised by filtration through a polyester membrane containing pores of diameter 0.22$\mu$m. Thus in a preferred aspect of the invention the pharmaceutical compositions are sterile.

Example 1 - Oil-in-Water Emulsion Ointment Composition

The percentage composition of the ointment is as follows:

| | % (w/w) |
|---|---|
| 2,4,4$^1$-Trichloro-2$^1$-hydroxydiphenyl ether | 2.0 |
| Dibromopropamidine isoethionate | 0.2 |
| Liquid paraffin | 6.0 |
| Cetostearyl alcohol | 7.2 |
| Cetomacrogol | 1.8 |
| White soft paraffin | 15.0 |
| Glycerol | 5.0 |
| Water | to 100.0 |

The water for example, distilled water, which had been pre-heated to 80°C is added to a vessel. The diphenyl ether and dibromopropamidine salt are mixed with the glycerol to form a smooth homogenous suspension and this is then added to the stirred water in the mixing vessel.

The alcohols and paraffin are melted together and heated to 70°C. The molten mixture is added to the aqueous component with stirring. Initially stirring is vigorous but as the mixture cools and thickens the rate of stirring is reduced.

The ointment is sterile and may then be poured into presterilised containers such as cylindrical pots or plastic tubes.

Example 2 - Aqueous Gel Composition

An aqueous gel was prepared which had the following composition.

| | |
|---|---|
| Hydroxyethyl cellulose | 2.0% |
| Propylene glycol | 10.0% |
| Dibromopropamidine isoethionate | 0.2% |
| 2, 4, 4$^1$-Trichloro-2$^1$-hydroxydiphenyl ether | 2.0% |
| Distilled water | to 100.0% |

The hydroxyethyl cellulose (2g) was dissolved with stirring in a major portion of the distilled water (80g). The dibromopropamidine salt 0.25g and 2, 4, 4$^1$-trichloro-2$^1$-hydroxydiphenyl ether (2.0)g were dispersed in propylene glycol (10g) to form a homogenous suspension. This suspension was added to the hydroxyethyl cellulose solution with stirring to provide a homogenous gel mixture and finally the weight of the gel was adjusted to 100g by addition of further distilled water.

The gel may be packaged in a similar manner to that described in Example 1.

Example 3 - Hydrophobic Ointment Composition

| | |
|---|---|
| 2, 4, $4^1$-Trichloro-$2^1$-hydroxydiphenyl ether | 2.0% |
| Dibromopropamidine isoethionate | 0.2% |
| Liquid paraffin/White soft paraffin (1:4 mixture) | to 100% |

The paraffin components are melted together and thoroughly mixed. The diphenylether and dibromopropamidine salt are added and the mixture stirred until it is homogenous. The ointment composition is then allowed to cool and the ointment may be packaged in a similar manner to that described in Example 1.

Example 4 - Pessary Composition

A pessary is prepared which has the following composition,

| | |
|---|---|
| 2, 4, $4^1$-Trichloro-$2^1$-hydroxydiphenyl ether | 200mg |
| Dibromopropamidine isoethionate | 20mg |
| Massa estarinium B | to 4g |

Example 5 - Oil-in-water Emulsion Ointment Composition

An ointment was prepared in a similar manner to that described in Example 1 except that the ointment contained 2, 4, $4^1$-trichloro-$2^1$-hydroxydiphenyl ether and dipropamidine 0.15% as the active ingredients.

Example 6 - Oil-in-water Emulsion Ointment Composition

An ointment was prepared in a similar manner to that described in Example 1 except that the ointment contained 2, $2^1$-dihydroxy-3, $3^1$, 5, $5^1$, 6, $6^1$-hexa chlorodiphenylmethane (2.0%) and dibromopropamidine isothionate (0.2%) as the active ingredients.

Example 7 - Oil-in-water Emulsion Ointment Composition

An ointment was prepared in a similar manner to that described in Example 1 except that the ointment contained 2, $2^1$-dihydroxy-3, $3^1$, 5, $5^1$, 6, $6^1$-hexachlorodiphenylmethane (2.0%) and propamidine (0.25%) as the active ingredients.

Example 8 - Preoperative Skin Scrub

| | |
|---|---|
| 2, 4, $4^1$-Trichloro-$2^1$-hydroxydiphenyl ether | 2.0% |
| Dibromopropamidine | 0.25% |
| Sodium lauryl ethoxy sulphate | 30.0% |
| Cococut diethanolamide | 2.0% |
| Anionic surfactant | 5.0% |
| EDTA (disodium salt) | 0.5% |
| Ethanol | 10.0% |
| Phosphoric acid to adjust to | pH 4.5 |
| Water | to 100% |

The dibromopropamidine, sodium lauryl ethoxysulphate, EDTA (disodium salt) and coconut diethanolamide were dissolved in water and the diphenyl ether dissolved in ethanol was slowly added with stirring. The pH was adjusted and the composition adjusted to the final weight with water.

Example 9 - Antiseptic Solution

The composition of an antiseptic solution is as follows,

| | |
|---|---|
| 2, 4, $5^1$-Trichloro-$2^1$-hydroxydiphenyl ether | 2.5% |
| Dibromopropamidine | 0.2% |
| EDTA (disodium salt) | 0.5% |
| Isopropanol | 70% |
| Glycerol | 10% |
| Water | to 100% |

The dibromopropamidine and EDTA (disodium salt) were dissolved in deionised water (15ml). The diphenyl ether was dissolved in a mixture of isopropanol and glycerol. This solution was slowly added with stirring to the aqueous solution. The final weight of the composition was adjusted to 100g by addition of water.

Example 10 - Oil-in-Water Emulsion Ointment composition

The composition of the ointment is as follows:

| | |
|---|---|
| 2, 4, $4^1$-Trichloro-$2^1$-hydroxydiphenyl ether | 2.0% |
| Dibromopropamidine isoethionate | 0.2% |
| Liquid paraffin | 6.0% |
| Cetostearyl alcohol | 7.0% |
| Cetomacrogol | 2.0% |
| White soft paraffin | 15.0% |
| Glycerol | 5.0% |
| EDTA (Disodium salt) | 0.5% |
| Water | to 100% |

The ointment was prepared in a similar manner to that described in Example 1.

Example 11 - Oil-in-Water Emulsion Ointment Composition

The composition of the ointment is as follows:

| | |
|---|---|
| 2, 4, $4^1$-Trichloro-$2^1$-hydroxydiphenyl ether | 2.5% |
| Hexamidine | 0.25% |
| Liquid paraffin | 6.5% |
| Cetostearyl alcohol | 6.5% |
| Cetomacrogol | 2.0% |
| White soft paraffin | 15.0% |
| Glycerol | 7.5% |
| EDTA (Disodium salt) | 0.5% |
| Water | to 100% |

Demonstration of Effectiveness

The minimum inhibitory concentration (MIC) of triclosan and dibromopropamidine individually and in combination were obtained against a range of test organisms, such as two different strains of Pseudomonas, a strain of Proteus and a strain of Serratia. The test method employed was as follows, a 2% solution of triclosan was prepared in 60% propylene glycol and than a range of dilutions made in distilled water. A solution of dibromopropamidine was prepared at a range of dilutions in water. The agents were then tested separately or in combination. Isosensitest (trade mark) broth was inoculated with a test organism at a concentration of $10^5$ organisms per ml and a sample was dispensed into wells of a microtitre plate. The range of concentration of test substance or combination was added to the wells so that there was a further 10-fold dilution of the test substance or combination. The plates were incubated for either 24 or 48 hours at 37° C, and the MIC read as the lowest concentration giving no visible growth. For each organism tested all

conditions of inoculum preparation, inoculation and incubation were standardised throughout.

The results of the tests given in Table 1 show synergy between triclosan and dibromopropamide.

TABLE 1

| Minimum inhibitory concentration (MIC) of 2,4,4[1]-trichloro-2[1]-hydroxydiphenyl ether (Triclosan) and dibromopropamidine both alone and in combination, against a range of organisms. | | | | |
|---|---|---|---|---|
| Organism | MIC : Individual Agents ($\mu g\ ml^{-1}$) | | MIC : Combination of Agents ($\mu g\ ml^{-1}$) | |
| | Triclosan | Dibromopropamidine | Triclosan | Dibromopropamidine |
| Proteus morganii (23 UWIST) | 2000 | 250 | 125 | 62.5 |
| Serratia marcescens | >2000 | 500 | 125 | 62.5 |
| Pseudomonas 4039 | 2000 | 500 | 15.6 | 250 |
| Pseudomonas aeruginosa (NC1B 8626) | 2000 | 500 | 62.5 | 250 |

The kill curve data for 2, 4, 4[1]-trichloro-2[1]-hydroxyldiphenyl ether and dibromopropamidine both alone and in combination were measured against a range of microorganisms. The antimicrobial agents were dissolved in 15% v/v propylene glycol and challenged by inoculation of the test bacteria at a concentration of approximately $10^6$ organisms per ml. The numbers of bacteria remaining after a given time were measured by removing aliquots from the solution, neutralising the antibacterial agent and estimating the number of surviving organisms in a conventional plate technique. The results illustrate the synergy between triclosan and dibromopropamidine at a weight ratio of the two components of 13:1 approx. and are shown in Table 2.

Table 2

0.2% (w/v) Triclosan + 0.015% Dibromopropamidine.

| Organism | Time (min after inoculation) 0 | 10 | 20 | 30 | 60 |
|---|---|---|---|---|---|
| Serratia marcescens | 5.97 | 3.13 | 2.84 | 2.94 | 2.95 |
| Pseudomonas aeruginosa (NC1B 8626) | 6.04 | 3.76 | 3.0 | <2.0 | <2.0 |
| Staphylococcus aureus | <2.0 | <2.0 | <2.0 | <2.0 | <2.0 |
| **0.2% (w/v) Triclosan** | | | | | |
| Serratia marcescens | 6.72 | 5.39 | 5.52 | 5.31 | 5.07 |
| Pseudomonas aeruginosa (NC1B 8626) | 6.49 | 6.20 | 6.12 | 5.98 | 5.97 |
| Staphylococcus aureus | <2.0 | <2.0 | <2.0 | <2.0 | <2.0 |
| **0.015% (w/v) Dibromopropamidine** | | | | | |
| Serratia marcescens | 6.64 | 6.50 | 6.04 | 5.20 | 3.98 |
| Pseudomonas aeruginosa (NC1B 8626) | 6.30 | 6.40 | 6.10 | 5.60 | 4.90 |
| Staphylococcus aureus | 6.73 | 4.70 | 6.71 | 6.44 | 5.58 |
| **15% (w/v) Propylene glycol** | | | | | |
| Serratia marcescens | 6.62 | - | - | - | 6.50 |
| Pseudomonas aeruginosa (NC1B 8626) | 6.20 | - | - | - | 6.48 |
| Staphylococcus aureus | 6.79 | - | - | - | 6.84 |

## Claims

1. A pharmaceutical composition suitable for topical application which comprises hydroxy halogenated diphenyl derivative of Formula (I)

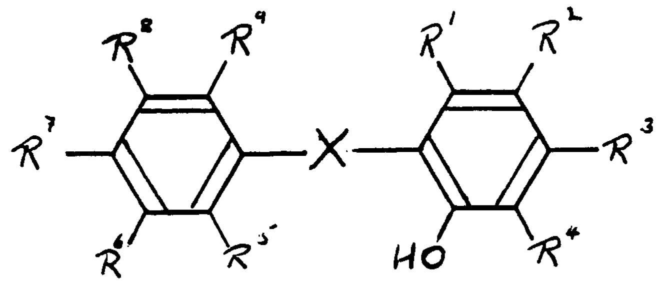

(I).

wherein X is O or $CH_2$ and each of $R^1$ to $R^9$ may be hydrogen, hydroxyl or halogen provided at least one of $R^1$ to $R^9$ is halogen and a diamidine of Formula (II)

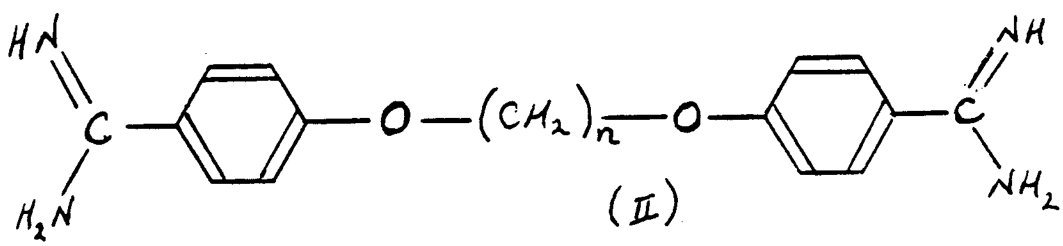

(II)

or a pharmaceutically acceptable salt thereof, wherein, n is an integer from 1 to 6 and each phenyl group may be further substituted by halogen, and a pharmaceutically acceptable carrier therefor.

2. A pharmaceutical composition as claimed in claim 1 in which the ratio by weight of the hydroxy halogenated diphenyl derivative of Formula (I) to diamidine of Formula (II) is in the range 25:1 to 1:25.

3. A pharmaceutical composition as claimed in claim 1 in which X is O and $R^1$ to $R^9$ are hydrogen or halogen in which at least two of $R^1$ to $R^9$ are chloro.

4. A pharmaceutical composition as claimed in claim 3 in which the diphenyl derivative of Formula (I) is 2,4,4$^1$-trichloro-2$^1$-hydroxydiphenyl ether.

5. A pharmaceutical composition as claimed in claim 1 in which X is $CH_2$ and $R^5$ is hydroxy and the remaining substituents are hydrogen or halogen.

6. A pharmaceutical composition as claimed in claim 5 in which there are at least two halogen substituents to each phenyl ring.

7. A pharmaceutical composition as claimed in claim 6 in which the diphenyl derivative of Formula (I) is 2,2$^1$-dihydroxy-3,3$^1$,5,5$^1$,6,6$^1$-hexachlorodiphenylmethane

8. A pharmaceutical composition as claimed in claim 1 in which the diamidine of Formula (II) is propamidine or a pharmaceutically acceptable salt thereof.

9. A pharmaceutical composition as claimed in claim 1 in which the diamidine of Formula (II) is dibromopropamidine or a pharmaceutically acceptable salt thereof.

10. A pharmaceutical composition as claimed in claim 1 which contains from 0.1 to 10% by weight of a diphenyl derivative of Formula (I).

11. A pharmaceutical composition as claimed in claim 1 which contains from 0.05 to 2% by weight of a diphenyl derivative of Formula (I) whereby the composition is to be applied to intact skin.

12. A pharmaceutical composition as claimed in claim 1 which contains from 0.01 to 1% by weight of a diamidine of Formula (II).

13. A pharmaceutical composition as claimed in any one of claims 1 to 12 in which the composition additionally contains 0.01 to 5% of ethylene diamine tetra-acetic acid or a salt thereof.

14. A pharmaceutical composition as claimed in claim 13 in which the composition contains from 0.05 to 2.5% by weight of the disodium salt of ethylene diamine tetra-acetic acid.

15. A pharmaceutical composition as claimed in any one of claims 1 to 14 wherein the composition is in the form of an ointment.

16. A pharmaceutical composition as claimed in any one of claims 1 to 14 wherein the composition is in the form of an aqueous gel.

17. A pharmaceutical composition as claimed in any one of claims 1 to 14 wherein the composition is in the form of a pre-surgical scrubbing composition which additionally contains alkanol.

18. A pharmaceutical composition suitable for topical administration for the treatment of burns, ulcers acne and other skin lesions exposed to the risk of infection comprises from 0.5 to 3% by weight of hydroxy halogenated diphenyl derivative of Formula (I) and 0.1 to 0.3% by weight of a diamidine of Formula (II) or a pharmaceutically acceptable salt thereof, 15 to 25% by weight of liquid paraffin, 7 to 15% by weight of polyhydric alcohol, 4 to 8% by weight of stearyl alcohol, 4 to 8% by weight of glycerol monostearate, 2 to 6% by weight of a non-ionic surfactant and water to adjust the weight to 100%.

**Patentansprüche**

1. Zur topischen Anwendung geeignete pharmazeutische Zusammensetzung, welche ein halogeniertes Hydroxydiphenyl-Derivat der Formel (I)

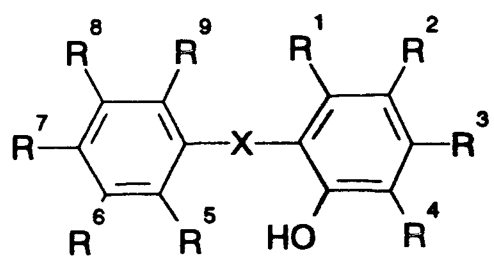

(I)

worin X O oder $CH_2$ ist und jedes von $R^1$ bis $R^9$ Wasserstoff,Hydroxy oder Halogen sein kann, vorausgesetzt wenigstens eines von $R^1$ bis $R^9$ ist Halogen, und ein Diamidin der Formel (II)

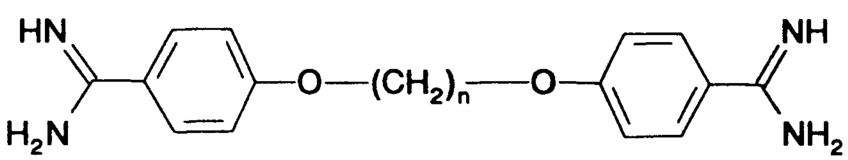

(II)

oder dessen pharmazeutisch annehmbares Salz, worin n eine ganze Zahl von 1 bis 6 ist und jede Phenyl-Gruppe durch Halogen weiter substituiert sein kann, und einen pharmazeutisch annehmbaren Träger hierfür umfaßt.

2. Pharmazeutische Zusammensetzung wie in Anspruch 1 beansprucht, in welcher das Gewichtsverhältnis des halogenierten Hydroxydiphenyl-Derivats der Formel (I) zum Diamidin der Formel (II) im Bereich von 25:1 bis 1:25 liegt.

3. Pharmazeutische Zusammensetzung wie in Anspruch 1 beansprucht, in welcher X O ist und $R^1$ bis $R^9$ Wasserstoff oder Halogen sind und in welcher wenigstens zwei von $R^1$ bis $R^9$ Chlor sind.

4. Pharmazeutische Zusammensetzung wie in Anspruch 3 beansprucht, in welcher das Diphenyl-Derivat der Formel (I) 2,4,4$^1$-Trichlor-2$^1$-hydroxydiphenylether ist.

5. Pharmazeutische Zusammensetzung wie in Anspruch 1 beansprucht, in welcher X $CH_2$ ist und $R^5$ Hydroxy ist und die verbleibenden Substituenten Wasserstoff oder Halogen sind.

6. Pharmazeutische Zusammensetzung wie in Anspruch 5 beansprucht, in welcher sich wenigstens zwei Halogen-Substituenten an jedem Phenyl-Ring befinden.

7. Pharmazeutische Zusammensetzung wie in Anspruch 6 beansprucht, in welcher das Diphenyl-Derivat der Formel (I) 2,2$^1$-Dihydroxy-3,3$^1$,5,5$^1$,6,6$^1$-hexachlordiphenylmethan ist.

8. Pharmazeutische Zusammensetzung wie in Anspruch 1 beansprucht, in welcher das Diamidin der Formel (II) Propamidin oder dessen pharmazeutisch annehmbares Salz ist.

9. Pharmazeutische Zusammensetzung wie in Anspruch 1 beansprucht, in welcher das Diamidin der Formel (II) Dibrompropamidin oder dessen pharmazeutisch annehmbares Salz ist.

10. Pharmazeutische Zusammensetzung wie in Anspruch 1 beansprucht, welche 0,1 bis 10 Gew.-% eines Diphenyl-Derivates der Formel (I) enthält.

11. Pharmazeutische Zusammensetzung wie in Anspruch 1 beansprucht, welche 0,05 bis 2 Gew.-% eines Diphenyl-Derivates der Formel (I) enthält, wobei die Zusammensetzung auf unversehrter Haut angewendet wird.

12. Pharmazeutische Zusammensetzung wie in Anspruch 1 beansprucht, welche 0,01 bis 1 Gew.-% eines Diamidins der Formel (II) enthält.

13. Pharmazeutische Zusammensetzung wie in irgendeinem der Ansprüche 1 bis 12 beansprucht, in welcher die Zusammensetzung zusätzlich 0,01 bis 5% Ethylendiamintetraessigsäure oder deren Salz enthält.

14. Pharmazeutische Zusammensetzung wie in Anspruch 13 beansprucht, in welcher die Zusammensetzung 0,05 bis 2,5 Gew.-% des Natriumsalzes von Ethylendiamintetraessigsäure enthält.

15. Pharmazeutische Zusammensetzung wie in irgendeinem der Ansprüche 1 bis 14 beansprucht, worin die Zusammensetzung in Form einer Salbe vorliegt.

16. Pharmazeutische Zusammensetzung wie in irgendeinem der Ansprüche 1 bis 14 beansprucht, worin die Zusammensetzung in Form eines wäßrigen Gels vorliegt.

17. Pharmazeutische Zusammensetzung wie in irgendeinem der Ansprüche 1 bis 14 beansprucht, worin die Zusammensetzung in Form einer prächirurgischen Scheuerzusammensetzung vorliegt, welche zusätzlich Alkohol enthält.

18. Eine zur topischen Verabreichung zur Behandlung von Verbrennungen, Ulcera, Akne und anderen, einem Infektionsrisiko ausgesetzten Hautläsionen geeignete pharmazeutische Zusammensetzung umfaßt 0,5 bis 3 Gew.-% halogeniertes Hydroxydiphenyl-Derivat der Formel (I) und 0,1 bis 0,3 Gew.-% eines Diamidins der Formel (II) oder dessen pharmazeutisch annehmbares Salz, 15 bis 25 Gew.-% Paraffinöl, 7 bis 15 Gew.-% eines mehrwertigen Alkohols, 4 bis 8 Gew.-% Stearylalkohol, 4 bis 8 Gew.-% Glycerin-monostearat, 2 bis 6 Gew.-% eines nichtionischen Tensids and Wasser, um das Gewicht auf 100% einzustellen.

**Revendications**

**1.** Composition pharmaceutique convenable pour l'application topique, qui comprend un dérivé hydroxy diphénylique halogéné de formule (I):

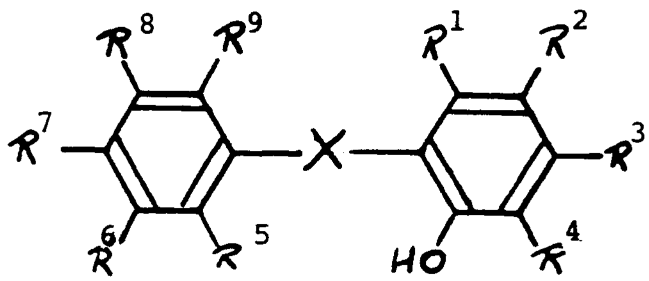

(I)

dans laquelle X est un atome d'oxygène ou un radical $CH_2$ et chacun des $R^1$ à $R^9$ peut être un atome d'hydrogène, d'halogène ou un groupe hydroxy à condition qu'au moins l'un des $R^1$ à $R^9$ soit un atome d'halogène,
et une diamidine de formule (II):

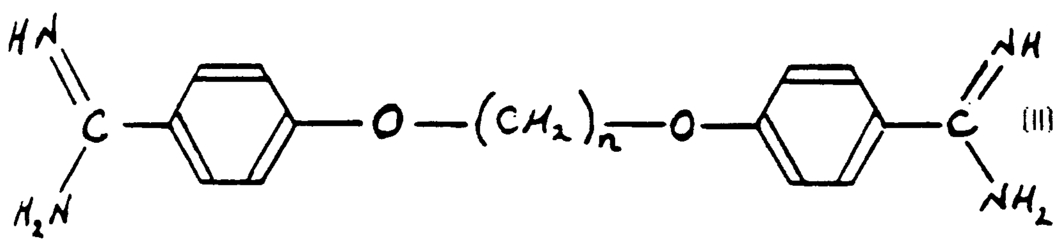

(II)

ou un sel de celle-ci acceptable du point de vue pharmaceutique, dans laquelle n est un entier de 1 à 6 et chaque groupe phényle peut être de plus substitué par un atome d'halogène,
et un support pour ceux-ci acceptable du point de vue pharmaceutique.

**2.** Composition pharmaceutique suivant la revendication 1, dans laquelle le rapport en poids du dérivé hydroxy diphénylique halogéné de formule (I) à la diamidine de formule (II) est dans la gamme de 25/1 à 1/25.

**3.** Composition pharmaceutique suivant la revendication 1, dans laquelle X est un atome d'oxygène et $R^1$ à $R^9$ sont un atome d'hydrogène ou d'halogène, deux au moins des $R^1$ à $R^9$ étant un atome de chlore.

**4.** Composition pharmaceutique suivant la revendication 3, dans laquelle le dérivé diphénylique de formule (I) est l'éther 2,4,4'-trichloro-2'-hydroxydiphénylique.

**5.** Composition pharmaceutique suivant la revendication 1, dans laquelle X est un radical $CH_2$ et $R^5$ est un groupe hydroxy et les substituants restants sont des atomes d'hydrogène ou d'halogène.

**6.** Composition pharmaceutique suivant la revendication 5, dans laquelle il y a au moins deux substituants halogène dans chaque cycle phényle.

**7.** Composition pharmaceutique suivant la revendication 6, dans laquelle le dérivé diphénylique de formule (I) est le 2,2'-dihydroxy-3,3',5,5',6,6'-hexachlorodiphénylméthane.

**8.** Composition pharmaceutique suivant la revendication 1, dans laquelle la diamidine de formule (II) est la propamidine ou un sel de celle-ci acceptable du point de vue pharmaceutique.

**9.** Composition pharmaceutique suivant la revendication 1, dans laquelle la diamidine de formule (II) est la dibromopropamidine ou un sel de celle-ci acceptable du point de vue pharmaceutique.

**10.** Composition pharmaceutique suivant la revendication 1, qui contient de 0,1 à 10% en poids d'un dérivé

diphénylique de formule (I).

**11.** Composition pharmaceutique suivant la revendication 1, qui contient de 0,05 à 2% en poids d'un dérivé diphénylique de formule (I), la composition devant être appliquée sur une peau intacte.

**12.** Composition pharmaceutique suivant la revendication 1, qui contient de 0,01 à 1% en poids d'une diamidine de formule (II).

**13.** Composition pharmaceutique suivant l'une quelconque des revendications 1 à 12, qui contient de plus 0,01 à 5% en poids d'acide éthylène diamine tétraacétique ou d'un sel de celui-ci.

**14.** Composition pharmaceutique suivant la revendication 13, qui contient de 0,05 à 2,5% en poids du sel disodique de l'acide éthylène diamine tétraacétique.

**15.** Composition pharmaceutique suivant l'une quelconque des revendications 1 à 14, qui est sous la forme d'une pommade.

**16.** Composition pharmaceutique suivant l'une quelconque des revendications 1 à 14, qui est sous la forme d'un gel aqueux.

**17.** Composition pharmaceutique suivant l'une quelconque des revendications 1 à 14, qui est sous la forme d'une composition de lavage pré-chirurgicale contenant de plus un alcanol.

**18.** Composition pharmaceutique convenable pour l'administration topique pour le traitement de brûlures, d'ulcères, d'acné et d'autres lésions cutanées exposées au risque d'infection, qui comprend de 0,5 à 3% en poids d'un dérivé hydroxy diphénylique halogéné de formule (I) et de 0,1 à 0,3% en poids d'une diamidine de formule (II) ou d'un sel de celle-ci acceptable du point de vue pharmaceutique, 15 à 25% en poids de paraffine liquide, 7 à 15% en poids d'alcool polyhydrique, 4 à 8% en poids d'alcool stéarylique, 4 à 8% en poids de monostéarate de glycérol, 2 à 6% en poids d'un tensioactif non ionique et de l'eau pour ajuster le poids à 100%.